# EUROPEAN PATENT SPECIFICATION

(11) **EP 4 543 533 B1**
(45) Date of publication and mention of the grant of the patent: **11.03.2026**
(21) Application number: 23745627.2
(22) Date of filing: 21.06.2023
(51) Int. Cl.: A61N 1/04, A61N 1/36, A61F 7/00, A61H 7/00, A61F 7/02, A61H 11/02, A61H 23/02

(54) **SKIN-TIGHT ELASTIC GARMENT FOR TRANSMISSION OF STIMULI TO A BODY**
HAUTDICHTES ELASTISCHES KLEIDUNGSSTÜCK ZUR ÜBERTRAGUNG VON REIZEN AUF EINEN KÖRPER
VÊTEMENT ÉLASTIQUE MOULANT POUR TRANSMISSION DE STIMULI AU CORPS

(30) Priority: 21.06.2022 PT 2022118059
(43) Date of publication of application: 30.04.2025
(73) Proprietor: CENTITVC - Centro de Nanotecnologia e Materiais Tecnicos, Funcionais e Inteligentes, 4760-034 Vila Nova de Famalicão (PT); CITEVE - Centro Tecnológico das Indústrias Têxtil e do Vestuário de Portugal, 4760-034 Vila Nova de Famalicão (PT); Universidade Do Porto, 4099-002 Porto (PT); Hata, Lda, Curvos, Esposende 4740-183 Curvos (PT); Tintex - Textiles, S.A., V.N. De Cerveira, 4920-009 Mentrestido (PT)
(72) Inventor: DA SILVA MIDÃO, Marta Sofia, 4760-034 VILA NOVA DE FAMALICÃO (PT); GONÇALVES DA ROCHA, Miguel Ângelo, 4760-034 VILA NOVA DE FAMALICÃO (PT); FRANCISCO GONÇALVES, Patrícia, 4760-034 VILA NOVA DE FAMALICÃO (PT); DE ARAÚJO SÁ, Isaque Joaquim, 4760-034 VILA NOVA DE FAMALICÃO (PT); SANTOS RAMALHO, Miguel, 4760-034 VILA NOVA DE FAMALICÃO (PT); FERNANDES DA SILVA ANDRADE LEITE, André Filipe, 4760-034 VILA NOVA DE FAMALICÃO (PT); DA COSTA MESQUITA, Rui Pedro, 4760-034 VILA NOVA DE FAMALICÃO (PT); FERREIRA DA SILVA, Juliana Sofia, 4760-034 VILA NOVA DE FAMALICÃO (PT); CORREIA DE OLIVEIRA BONIFÁCIO SOTTO MAYOR PIZARRO, Maria Da Graça, 4760-034 VILA NOVA DE FAMALICÃO (PT); PEREIRA VILAÇA, Maria Helena, 4760-034 VILA NOVA DE FAMALICÃO (PT); MORIM FIGUEIREDO RAMÔA, Ana Florinda, 4760-034 VILA NOVA DE FAMALICÃO (PT); VILAS-BOAS SOARES CAMPOS, João Paulo, 4200-450 PORTO (PT); PINTO FERNANDES, Ricardo Jorge, 4200-450 PORTO (PT); MARQUES DUARTE, João Pedro, 4200-450 PORTO (PT); ALVES MACHADO DE SOUSA, Filipa Manuel, 4200-450 PORTO (PT); RODRIGUES MACHADO, Leandro José, 4200-450 PORTO (PT); TORRE COUTO, Suse Mariana, 4740-183 CURVOS (PT); SILVA DE SOUSA, Nuno Ricardo, 4740-183 CURVOS (PT); REBELO ALHEIRA, Diana Solange, 4930-513 SÃO PEDRO DA TORRE (PT); GONÇALVES PIRES DA SILVA, Carlos Filipe, 4460-832 CUSTÓIAS MTS (PT); REGO DOS SANTOS, Rute Marisa, 4900-215 VIANA DO CASTELO (PT); MARTINS CARDOSO DE MAGALHÃES, Pedro José, 4900-215 VIANA DO CASTELO (PT)
(74) Representative: Patentree
(86) International application number: PCT/IB2023/056422
(87) International publication number: WO 2023/248155

(56) References cited:
- DE-U1- 202011 109 226
- US-A1- 2015 202 429
- US-A1- 2017 266 431

## Description

### TECHNICAL FIELD

The present disclosure relates to a skin-tight elastic garment comprising several sensor-actuator pads stimuli for improving muscular recovery. Particularly, it relates to leggings comprising a plurality of stimuli to improve the muscular recovery of the user.

### BACKGROUND

Nowadays, the practice of sports has shown positive implications for health, personal and social values, thereby increasing the population's adherence to this activity. Amateur athletes are increasingly looking to use high-tech materials that until recently were only available to professionals while constantly measuring their performance and analysing their health. Like professional athletes, amateur athletes also look for high levels of endurance, strength, and resilience. Whether athletes are competing as professionals, amateurs, or just trying to improve the personal record, having enough energy to confront physical training is essential. Unfortunately, many athletes are plagued by early fatigue, which hinders their performance and limits their potential. Research shows that fatigue is a natural response when the body depletes its resources or accumulates too many harmful substances, with various factors, such as poor nutrition, bodily changes, environmental factors, and underlying medical conditions, at the root of it. Thus, one of the main concerns related to sports practice is muscle recovery after exercise, which allows delay the subsequent feeling of muscle discomfort and fatigue. In addition to low-intensity exercise, electrostimulation, compression, massage, and heating are methods usually used for recovery after physical incitement.

The methods commonly used for muscle recovery are electrostimulation, heating, compression and massage, which are more efficient than passive recovery which is without the use of processes, means and devices.

Recovery by electrostimulation through fixed devices using vests is quite restrictive, as the size of the devices and body contact accessories are impractical to be transported, limiting the place of use to a single space. In the case of portable devices, the need to use cables limits the user's freedom of action and the limited number of channels per device restricts the body areas that can be operated at the same time, extending the intervention time. The weight of the cables (and the wireless device boxes) makes the self-adhesive electrodes peel off frequently, due to the action of the movements triggered by the programs, reducing the comfort of their use. The loss of adhesion to the skin by the electrodes, with few uses, implies moderately high costs in their replacement, as they cannot be washed, and their functionality decreases with use.

Regarding to massage, its main limitation is the usual dependence on a third person to perform it, normally requiring a prior appointment and a limited time for its performance. In addition, some techniques can be aggressive and uncomfortable for deeper benefits, or, using a more traditional compression, it ends up requiring the patient to remain in a sitting or lying position throughout the session. In the case of portable devices for domestic use, these are less effective, forcing the replacement of the accessories used, or their position, when it is intended to change the body area of action, making the process time-consuming.

Finally, the application of heat implies the use of accessories (such as bathtubs and saunas) that are too bulky and/or expensive, normally requiring travel to specific spaces and consequent prior appointment. In the case of devices with infrared or laser technology, it is necessary to resort to specialized spaces, which are even rarer for the amateur athlete. In the case of wet and hot compresses, they need a hydrocollector, which is extremely bulky, expensive, and not portable. In terms of more portable options, hot compresses can be used in a domestic environment, but they have a very small area of action, requiring a long time of use to cover different body areas. As an alternative, there are heating creams, although they are difficult to use in certain body areas, in addition to the discomfort of eliminating the respective waste.

The document US2015202429A1 discloses a device designed for muscular stimulation of the trunk and/or leg muscles through electromyostimulation, intended to enable patients to benefit from electrostimulation in a home environment. The device consists of an elastic fabric forming the garment, an electrical bus, and a plurality of sensor-actuator pads, each electrically connected to the electrical bus.

The document US2017266431A1 discloses a electrotherapeutic garment, for example in the form of pants, having at least one electrode portion and an electrically conductive tracer ribbon embedded in the garment for connecting the electrode portion(s) to an electro-stimulation device.

These facts are disclosed in order to illustrate the technical problem addressed by the present disclosure.

### GENERAL DESCRIPTION

The invention is defined by independent claim 1. The present description relates to a solution for athletes looking for easy-to-use solutions that help them overcome the fatigue problems they may experience and reach higher limits of physical performance. The present disclosure relates to a skin-tight elastic that can electro-stimulate, massage, compress and heat the user by using emerging technologies, including integrated electronics in textiles and printing electronic devices.

The present disclosure presents three types of systems, fully integrated into textile structures, namely:
- massage and compression systems, through the integration of shape memory materials; and/or
- electrostimulation systems, through the integration of conductive wires and/or printed electronics; and/or
- heating systems, by integrating conductive polymeric fibres and/or printed electronics.

Simultaneously, methods for evaluation and analysis can be developed, which will allow the establishment of recovery programs to be integrated into a monitoring and control unit. Considering flexibility and customization, the solution also comprises a mobile application to interact (via Bluetooth or other system) with the hardware system.

In an embodiment, the system is battery powered and can be recharged wirelessly.

The present disclosure relates to a skin-tight elastic garment for transmission of one or more stimuli to the skin, comprising:
an elastic fabric from which the garment is made of;
an electrical bus stitched over a longitudinal seam of the garment;
a plurality of sensor-actuator pads, each electrically coupled to said electrical bus.

A skin-tight garment is a garment that is held to the skin usually by elastic tension using some type of stretch fabric. Also referred as a second-skin garment, a tight-fit elastic garment, a compression/compressive elastic garment, a body shape-adaptive garment, or a form-fit garment.

Examples of a skin-tight elastic garment include leggings, bodysuits, stockings, skin-suit sportswear garments, tight-fitting sportswear shirts, tight-fitting thermal garments, among others.

In an embodiment, the sensor-actuator pads comprise an electrostimulation pad comprising:
a plurality of textile electrodes for contacting with the skin;
a foam between the fabric and the plurality of textile electrodes;
a plurality of connectors to connect the electrostimulation pad to the electrical bus.

The present disclosure also relates to a skin-tight elastic garment for transmission of one or more stimuli to the skin, comprising:
an elastic fabric from which the garment is made of;
an electrical bus stitched over a longitudinal seam of the garment;
a plurality of sensor-actuator pads, each electrically coupled to said electrical bus;
wherein each sensor-actuator pad of the plurality of sensor-actuator pads comprises an electrostimulation pad comprising
   a plurality of textile electrodes for contacting with the skin;
   a foam between the fabric and the plurality of textile electrodes;
   a plurality of connectors to connect the electrostimulation pad to the electrical bus.

In an embodiment, each textile electrode of the plurality of textile electrodes comprises a conductive coating on a surface arranged to face the skin.

In an embodiment, the conductive coating comprises a conductive polymer, in particular polydimethylsiloxane.

In an embodiment, the conductive coating comprises conductive carbon materials, in particular carbon black and/or nano-structured carbon, further in particular carbon nanotubes.

In an embodiment, the plurality of textile electrodes are conductive yarns sewn to an electrostimulation pad fabric, preferably the electrostimulation pad fabric being a knitted material.

In an embodiment, the conductive yarns have 117 dtex to 235 dtex.

In an embodiment, the plurality of textile electrodes is a fabric comprising a conductive ink.

In an embodiment, the conductive ink is a silver ink.

In an embodiment, the foam is a synthetic foam, preferably neoprene foam.

In an embodiment, the connectors are crimped connections.

In an embodiment, the sensor-actuator pads comprise a thermal pad comprising:
a polymeric fibre charged with conductive particles or a fabric with a conductive ink, and
thermal pad connectors for connecting conductive elements of the thermal pad to the electrical bus.

In an embodiment, the conductive ink is silver ink or the conductive particles are carbon black particles.

In an embodiment, the fabric with a conductive ink comprises polyurethane on a surface arranged to face the skin.

In an embodiment, the polymeric fibre comprise a sheath made of a combination of polyolefin and carbon material and a core made of polyolefin, in particular the polyolefin being polypropylene.

In an embodiment, the sensor-actuator pads comprise a compression pad comprising a textile elastic band with a shape-memory material and compression pad connectors for electrically connecting the compression pad to the electrical bus.

In an embodiment, the shape-memory material is a spring made of a shape-memory alloy.

In an embodiment, the compression pad further comprises rivets for connecting the springs to a non-elastic textile fabric for limiting extension of the spring.

In an embodiment, the shape-memory material is a fibre made of a shape-memory polymer.

In an embodiment, the device comprises an electronic control device for controlling the plurality of sensor-actuator pads.

In an embodiment, the device comprises a waist band for supporting the electronic control device.

In an embodiment, the device comprises electrical connectors for electrically connecting the waist band to the electrical bus.

The advantages of the shape memory alloys (SMA's) are: change in compression at command; possibility of massage, using each line of 4 SMA's; quieter then compression with pressotherapy; the SMA's can be used for both compression and heat treatment (joule effect); portable and lighter than other SMA's solutions; less power consumption in comparison to other SMA's solutions; easier to adjust to the users size than other SMA's solutions.

The advantages of the shape memory polymers (SMPs) are: easy processing; easy integration (integration by conventional textile processes); seamless solution (the fibres have a small dimension and are directly integrated in the leggings) and comfort.

The advantages of the printed heating circuit are: fully integrated in textile structure or fabric; does not affect textile flexibility; sufficient heating with 12 V; low-cost production process.

The advantages of the thermoplastic fibres for Joule heating are: low weight; possibility of integration by conventional textile processes; comfort and flexibility.

All the above-mentioned advantages make these fibres more suitable than conventional solutions (metallic wires).

The advantages of the textile electrostimulation electrodes are: fully integration in textile structure; can be washed; reduces skin breathing problems by avoiding irritations inherent to commercial hydrogel alternatives; more comfortable than hydrogel solutions; can be reused and the electrodes are dry, avoiding the need to add water for proper functioning, when comparing to the commercial textile solutions.

The advantages of the printed electrostimulation electrodes are: fully integrated in textile structure and does not affect textile flexibility.

The advantages of the electronics are: battery powered, wireless charging device and Bluetooth enabled device that offers control over three independent stimuli (Massage, Thermal or Heating and Electrostimulation). The user can interact with the device through an Android application.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of a jig for producing SMA spring.
**Figure 2****:** Schematic representation of an embodiment of a compression and massage pad.
**Figure 3****:** Schematic representation of an embodiment of the electrostimulation pads wherein 31 represents the polymer foam, preferably neoprene, 32 represents the embroidered electrode and 33 represents the conductive coating comprising of Polydimethylsiloxane (PDMS) and carbon material.

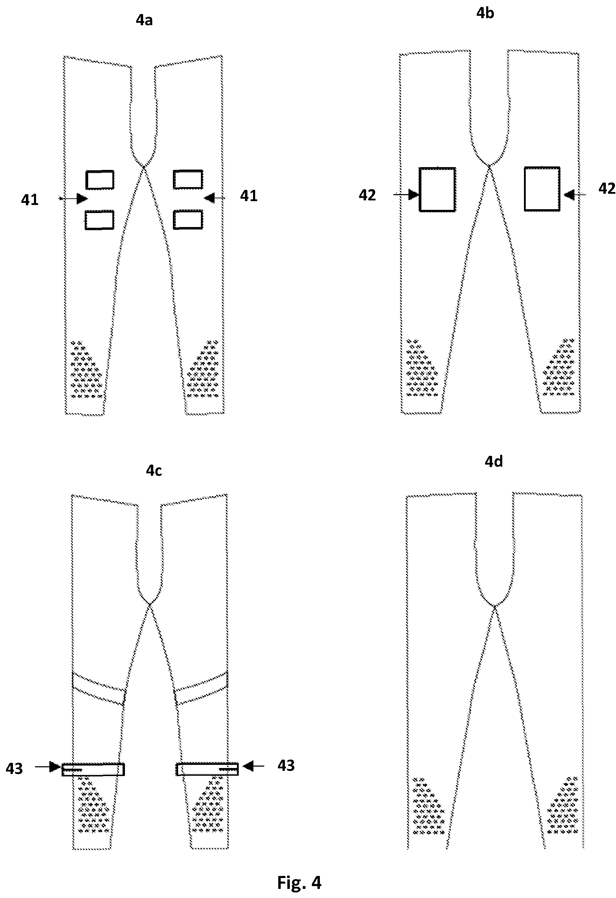

**Figure 4****:** Schematic representation of a simplified design an embodiment of the leggings (upper and middle), and where each system is preferably located, namely the thermal or heating is applied over hamstring muscles; electrostimulation is applied over quadricep muscles; massage and compression is applied over gastrocnemius muscles wherein **4a** represents the inside-front of the leggings, **4b** is inside-back of the leggings, **4c** is the outside-front of the leggings and **4d** is the outside-back of the leggings, **41** is electrostimulation sensors, **42** is heating sensors and **43** is SMAS.
**Figure 5****:** Schematic representation of an embodiment of the electronic system.
**Figure 6****:** Schematic representation of an embodiment of the electronic control device.
**Figure 7****:** Schematic representation of the interaction between the device and the user.
**Figure 8****:** Schematic representation of an embodiment indicating location of each sensor-actuator pad wherein **A** is control electronics and battery, **B** electrostimulation pad, **C** heating pad, **D** massage and compression pad and **E** is Bluetooth controlled device via mobile application.

### DETAILED DESCRIPTION

The present disclosure relates to a skin-tight elastic garment able to produce different stimulus to a body of a user. It relates to a skin-tight elastic garment for transmission of one or more stimuli to a body, comprising an elastic fabric and: an electrical bus stitched over a longitudinal seam of the leggings: a plurality of sensor-actuator pads, each electrically coupled to said electrical bus.

The present disclosure relates to a skin-tight elastic garment for transmission of one or more stimuli to the skin, comprising: an elastic fabric from which the garment is made of; an electrical bus stitched over a longitudinal seam of the garment; a plurality of sensor-actuator pads, each electrically coupled to said electrical bus.

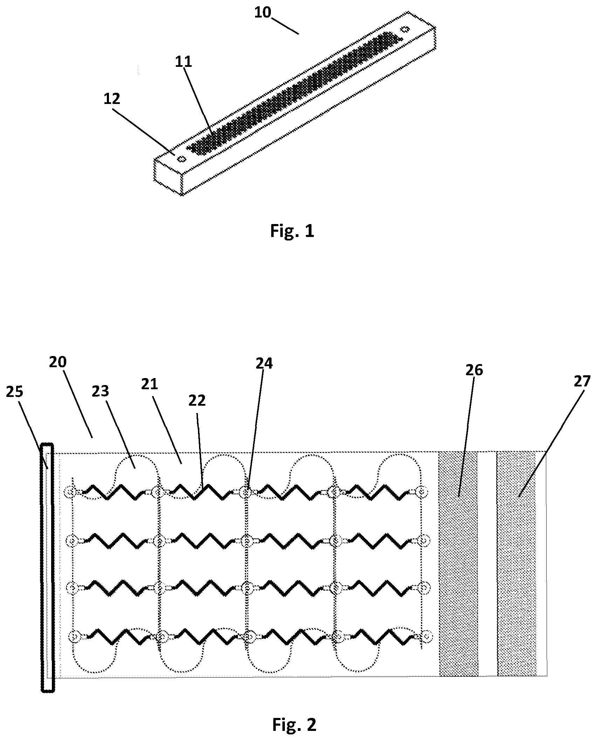

In an embodiment, for massage and compression, the shape memory alloy, SMA's springs were obtained from a commercial NiTi (nitinol wire) with an activation temperature of 55 °C. This wire is an equiatomic alloy of nickel and titanium. The wire is round-shaped and has 0.5 mm diameter. To produce these springs, 2 steel jigs were used. The technical drawings of these jigs can be seen in **Figure 1**. The jig **10** comprises a metal plate **12** and rods **11**.

The wire is wound up around stainless-steel metal rods with 1.8 mm diameter between two of these metal plates. At each turn another rod is placed to make the next turn, while keeping a manual tension. The two metal plates are then joined together with screws (e.g. 2 M3x15 screws) and nuts (e.g. M3 nuts).

After this, the spring and jig are placed inside a non-ventilated high temperature oven at 420 °C for 5 minutes. Then a quenching is done in a bucket with water and ice (approximately 0 °C).

Finally, the spring is removed by unscrewing the M3X15 screws and sliding the metal rods out. The width of the final spring is 6.2 mm.

Then the springs are cut and crimped with a ring terminal, leaving an active area of 4.5 cm.

This ring is fixed to a textile band, preferably composed by pine bark, with rivets that assure both the electrical and mechanical contact. Preferably the textile band is made of knitting. The maximum extension of each spring (6.3 cm) is assured by the placement of an inextensible textile. The representative drawing of this implementation is shown in **Figure 2****.**

**Figure 2** is a representation of an embodiment of the compression and massage pad **20**; with an elastic textile **21**; **22** is the spring; **23** is non-elastic fabric joining rivets **24**; **25** is a buckle or clasp; **26** and **27** are loop hook faster.

In an embodiment, the textile band is a knitting comprising lyocel, polyamide and elastane. Preferably, the textile band comprises 73% of lyocell, 24% of polyamide and 3% of elastane.

In an embodiment, for massage and compression, fibres were obtained from a commercial shape-memory polymer (SMP) with a glass transition temperature (Tg) of 55 °C. The SMP was produced by melt spinning process, using a multi-component fibre extruder from Hills Inc, Co. A 36 filaments spinneret with 0.6 mm of diameter was used. During the melt-spinning process, the fibres were stretched in a ratio of 2 to give the fibre a temporary shape. The obtained fibre has a linear density of 435 dtex.

In an embodiment, the fibres were integrated by braiding into a textile structure; a set of 4 fibres were braided together in 6 lines spaced by 10 mm to 15 mm, obtaining a compression band between 70 mm and 90 mm in length. This band was able to perform compression of 10 mmHg on the leg, using an external heating source for the SPM's fibres activation.

In an embodiment, the thermal pad or heating system is made with printed heating circuits. A conductive silver ink, preferably an ink with a viscosity (10,000 CPS) 10 Pa.s 25°C, and electrical resistance < 0.010 ohms/square for 25.4microns thickness, preferably a conductive ink by Engineered Materials Solutions, was printed by screen printing technique into a textile structure, preferably a knitted, more preferably comprising Lyocell, polyamide and elastane, previously treated with a coating, preferably a polyurethane based coating, to uniformize and block its surface. This coating guarantees a uniform surface for the silver ink application. In the printing process, a polyester 32.70 mesh with a typical bars pattern was used. This pattern allowed a differentiated heating in different areas of the circuit, with a higher temperature in the centre of the circuit and lower in the extremities. The printed circuits were than cured at 110°C for 15 minutes and a second cure was applied to fully process the silver ink with 100 °C for 30 minutes. Circuits with 15 Ω of electrical resistance were obtained in coated textile structures, allowing the centre area to reach more than 50 °C with a voltage of 12 V. The 2 electrical connections of the circuit were made using an anisotropic conductive film, with a resistance below 1 Ω, and copper wires to connect to the controlling electronics.

In an embodiment, to encapsulate the printed circuit and protect it to washing cycles, a 100 % polyurethane film was applied on top of the circuit by pressing with 140 °C and 4 bar for 20 seconds. This film also helps to protect the electrical connections of the circuit.

In an embodiment, the heating system or thermal pad can comprise thermoplastic fibres for Joule heating.

In an embodiment, a bicomponent fibres with a sheath/core (25/75, V/V) geometry were produced by melt spinning technique, using a multi-component fibre extruder preferably from Hills Inc, Co. It was used a filaments spinneret with 0.6 mm of diameter and a draw ratio of 1.2 was applied. The core of the fibre contains virgin polypropylene to improve mechanical properties and the sheath comprises a compound of polypropylene with 12.5 % Multi-Walled Carbon Nanotube (MWCNTs). Fibres with a linear density of 3580 dtex were obtained. To evaluate the joule effect the produced fibres were unidirectionally aligned in an acrylic fixture to achieve a compact substrate. Bus bars spaced by 6 cm were applied over this substrate transversely to the direction of the fibres. A voltage of 48 V was applied. A maximum temperature of 52.2 °C after 5 minutes was obtained. In an embodiment, the electrostimulation system is made with textile electrostimulation electrodes. To produce this textile-based electrodes for electrostimulation, square and rectangular electrodes with 5x5 cm and 5x8 cm respectively were embroidered by sewing (using e.g. lockstitch (single-step zig-zag conventional sew), chain stitch, two needle multi thread chain stitch) on a textile structure, preferably knitted material, more preferably comprising Lyocell, polyamide and elastane with the smallest possible spacing between yarns, preferably 0,05 cm and 0,1 cm, for assuring contact between yarns. These embroideries were prepared using a highly conductive embroidery yarn with 117 dtex to 235 dtex, preferably Shieldex from the supplier Statex.

To ensure an efficient electrostimulation a conductive coating comprising of Polydimethylsiloxane (PDMS) and 6 % - 9 % of carbon material was applied on top of the embroidered electrode. In the case of square embroidered electrodes, a coating measuring 5.5 cm x 5.5 cm was applied, while for the rectangular electrodes, a coating of 5.5 cm x 8.5 cm was applied. Finally, to ensure good adhesion of the conductive coating to the textile-based electrode, a constant force between 10 N and 30 N was applied to the coating for 2 days, ensuring its curing.

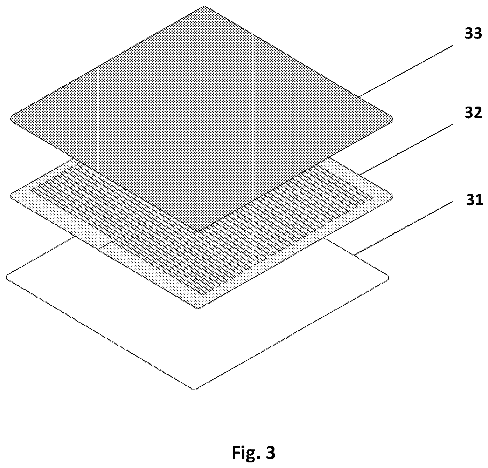

In an embodiment, **Figure 3** represents the electrostimulation pads comprising a foam **31,** preferably neoprene, the embroidered electrode **32** and the conductive coating comprising of Polydimethylsiloxane (PDMS) and carbon material **33.** Preferably the carbon material is carbon nanotubes.

In another embodiment, the electrostimulation can be done by printed electrostimulation electrodes. A conductive silver ink with viscosity 10,000 CPS 25°C, and electrical resistance < 0.010 ohms/square for 25.4 microns thickness, for example CI1036 by Engineered Materials Solution, was printed by screen printing technique onto a textile structure, preferably a knitted, previously treated with a coating, preferably a polyurethane based coating, to uniformize and block its surface. This coating is important to guarantee a uniform surface for the silver ink application. 2 electrodes were printed using a polyester 90.40 mesh directly onto the coated textile. To connect the electrode to the controlling electronics, a metallic crimp was inserted in the centre of each electrode and a copper wire was welded to this crimp.

To ensure an efficient electrostimulation a conductive coating composed of Polydimethylsiloxane (PDMS) and 6 % - 9% of carbon material was applied on top of the printed circuits.

In an embodiment, it is disclosed a skin-tight elastic garment, in particular leggings, an electronic control system, and a mobile application. For the leggings with the different solutions integrated, these are composed of two layers of the same knitted fabric, preferably a knitted fabric comprising Lyocell, polyamide and elastane. The electronics are integrated in one of the layers, after cutting their patterns. The cables that establish the connection between each system and the control and battery pass along the legs between the two layers of knitted fabric, thus avoiding direct contact with the skin. One or more of the systems is integrated in the leggings described below, preferably a combination of A, B and C.
A) Massage and compression systems, through the integration of SMAs: the textile band with the springs attached by rivets was integrated in the outer side of the outer layer of the leggings, by attaching two pressure snap buttons on the leggings (on the external side of the leg) and on the textile band. The electronic connection of the SMAs textile band with the leggings (to establish connection with the control), was done using eye terminals. The cable that establishes the electronic contact between the massage and compression system and the control and battery contains an eye terminal, that is fixed to one of the pressure snap buttons, used to fix the textile band to the leggings, on the inner side. The adjustment of the textile band to the leg (more or less tight) is done by using two stripes (made on the textile band) with hook and loop fastener on one terminal of the band, and two multipurpose loops on the other terminal of the band.
B) Electrostimulation systems through the integration of textile electrodes: each of the coated textile-based electrodes described in the individual solution were integrated in the leggings by sewing (by for example lockstitch (single-step zig-zag conventional sew), chain stitch, two needle multi thread chain stitch); a frame, preferably made of the same knitted material as the leggings, to the inner side of the inner layer of these (with direct contact with the skin) in the targeted locations. This frame hold the coated textile-based electrode in the targeted location, with a neoprene foam (sized to fit within the frame) between the embroidered electrode (with the coating facing out out / the skin) and the legging's textile, to increase the contact between the electrode and the skin. The coated textile-based electrode was bonded to the frame using a thermo glue. To connect the textile-based electrode to the controlling electronics, a metallic wire ferrule was crimped to the conductive textile yarn and passed through the neoprene foam and knitted layer of the leggings (so that the cable could pass on the other side of the fabric), and a copper wire was soldered to this ferrule.
C) Heating systems as thermal pads, by the integration of conductive printed electronics: the conductive printed circuit with the PU film and electrical connections was bonded to the leggings by thermo glue (at least 1 cm width) and a sew (for example lockstitch (single-step zig-zag sew), chain stitch, two needle multi thread chain stitch) in the inner side of inner layer of the leggings (PU film to be in contact with the skin). The cables were passed through the fabric (through a small laser cut insert) to the other side.

The user interacts with the system through the developed mobile application which connects to a selected device via Bluetooth.

In an embodiment, the leggings developed have integrated textile electrodes, massage and heating bands and temperature sensors. These actuators and sensors are connected to the electronic system through a set of wires which are terminated into three separated connectors, one for the sensors, one for the right side of the leg and another one for the left side of the leg.

In an embodiment, the garment comprises a belt, composed of three layers: an exterior layers preferably of the same knitted material as the leggings for aesthetics purpose, and an interior layer of an inextensible textile, preferably composed by pine bark, for structural purposes. The adjustment and clasp of the belt is done by pressure snap buttons. The control system and the battery cases, which have an aperture on the backside, are attached in the belt by passing a strip of the inextensible textile through this aperture and are integrated, on opposite sides of the belt fixing this strip to the belt with at least two pressure snaps on each side. The electronics developed for this system independently control the three actuations' systems: the heating system, the massage system, and the electrostimulation system.

In an embodiment, the electronic system developed is battery powered and can be recharged by any wireless charger compatible with the Qi v1.2 standard (developed by the Wireless Power Consortium). The system can also be recharged by any wired charger capable of delivering a voltage of 5V and 1A, being the USB Type C port the implemented wired input.

The user, through the mobile application can select a therapy (or build one) that consists in predefined programs which activate the different stimuli provided by the leggings, such as electrostimulation, heating, or massage. These programs are parameterized, and the parameters differ among the stimuli. In the case of the massage system the user can define the zone and the duration of the stimulus. In the heating system the user can define the zone, the duration, and the temperature of the stimulus. In the electrostimulation the user can select the zone, the duration, and the intensity of electrostimulation.

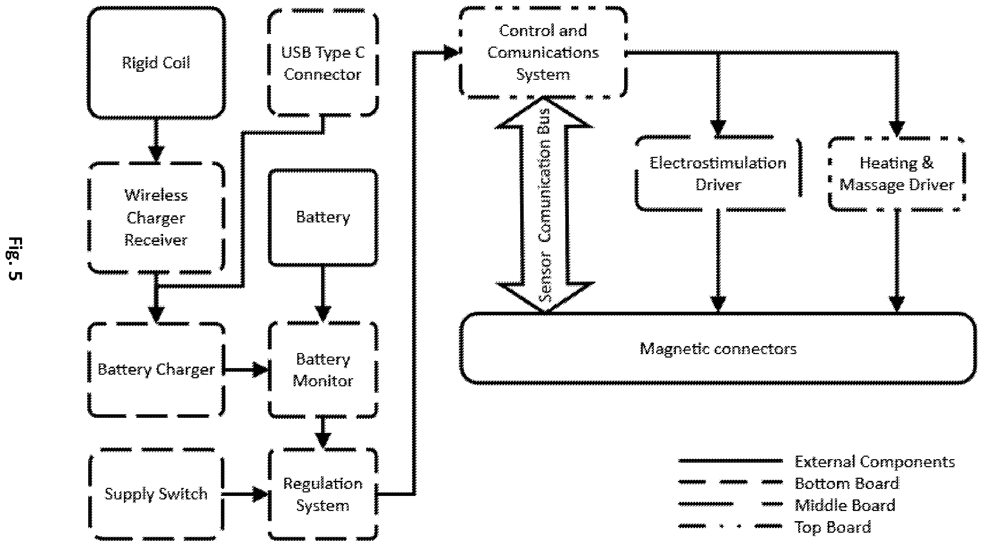

The developed electronics can be divided into three parts: the supply system, the control and communications system, and the drivers system (see **Figure 5**).

The supply system is responsible for the charging and monitoring the battery's state and it is also in this system where all the nominal voltages used in the forward systems are regulated. The control and communications system is responsible for controlling the stimuli via the drivers and establish a Bluetooth connection with a mobile device. The drivers system is composed by all the necessary components to control the regulated voltages required to apply the different stimuli. These parts are represented in the image below and its marking represent in which board they are allocated.

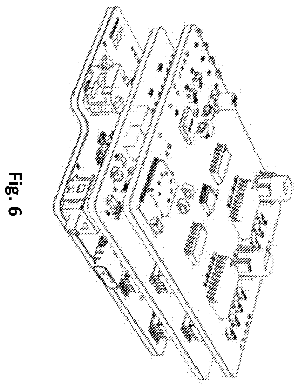

In an embodiment, the developed hardware is composed by three boards that accommodate all the above systems. These boards are connected between them, has illustrated in the **Figure 6**.

The user, through the mobile application, interacts with the developed device which is a Bluetooth enabled device. The mobile application make use of the BLE Stack, thus using its methods to communicate properly with the device.

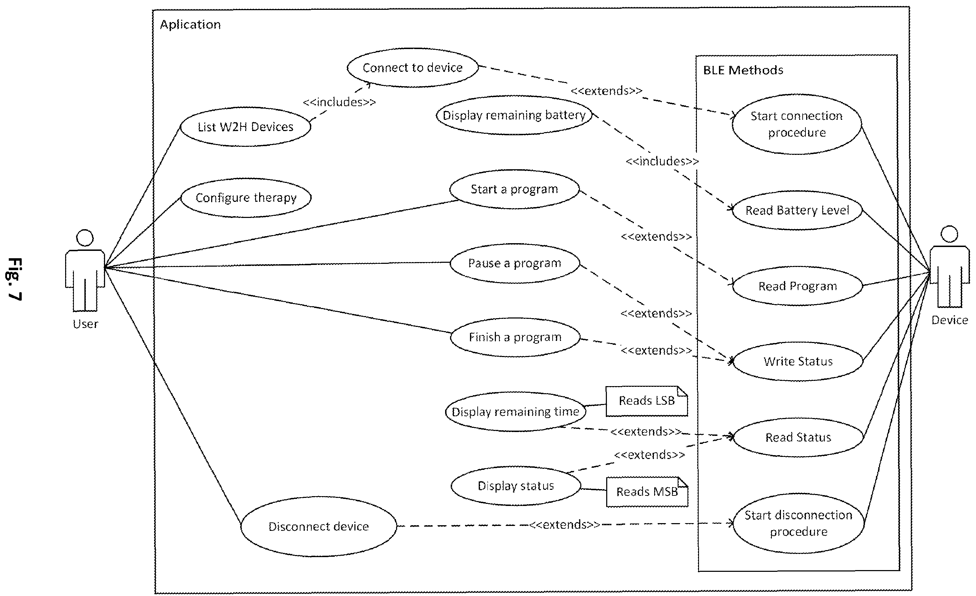

The developed mobile application enables the user to connect/disconnect with the device, configure therapies, as well as start, pause and stop the program of a determined therapy. The mobile application displays the current battery level of the connected device and can obtain the status of the device for controlling purposes (see **Figure 7****).**

**Figure 4** shows an embodiment of the leggings (upper and middle), where each sensor-actuated pad is preferably located. The thermal pad is applied over hamstring muscles; electrostimulation pad is applied over quadricep muscles; massage and compression pad is applied over gastrocnemius muscles.

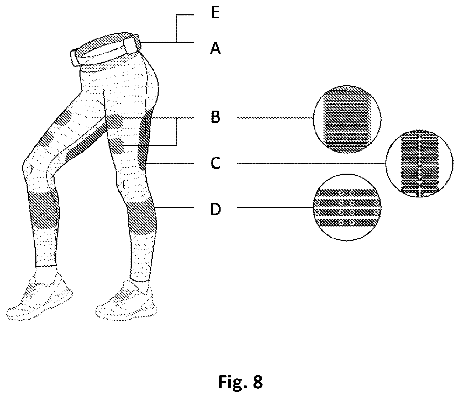

**Figure 8** also shows and embodiment comprising enclosures with control electronics and battery **A,** electrostimulation pad **B**; heating pad **C**; massage and compression pad **D**; and also is disclosed the Bluetooth controlled device via mobile application **E**.

This work was co-financed by Operational Program for Competitiveness and Internationalization (COMPETE 2020), under the PORTUGAL 2020 Partnership Agreement, through the European Regional Development Fund (ERDF).

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

## Claims

1. Skin-tight elastic garment (4) for transmission of one or more stimuli to the skin, comprising:
an elastic fabric (4a - 4d) from which the garment is made of;
an electrical bus (41) stitched over a longitudinal seam of the garment;
a plurality of sensor-actuator pads (B, C, D), each electrically coupled to said electrical bus (41);
wherein each sensor-actuator pad of the plurality of sensor-actuator pads comprises an electrostimulation pad (B) comprising
a plurality of textile electrodes (32) for contacting with the skin;
a foam (31) between the fabric (4a - 4d) and the plurality of textile electrodes (32);
a plurality of connectors (33) to connect the electrostimulation pad (B) to the electrical bus (41).

2. Garment (4) according to the previous claim wherein each textile electrode (32) of the plurality of textile electrodes comprises a conductive coating (33) on a surface arranged to face the skin.

3. Garment (4) according to claim 2 wherein the conductive coating (33) comprises a:
conductive polymer, in particular polydimethylsiloxane;
or carbon materials, in particular carbon black and/or nano-structured carbon,
further in particular carbon nanotubes.

4. Garment (4) according to any of the previous claims wherein the plurality of textile electrodes (32) are:
conductive yarns sewn to an electrostimulation pad fabric (B), preferably the electrostimulation pad fabric (B) being a knitted material;
in particular having 117 dtex to 235 dtex.

5. Garment (4) according to any of previous claims wherein the plurality of textile electrodes (32) is a fabric comprising a conductive ink.

6. Garment (4) according to any of the previous claims wherein:
the foam (31) is a synthetic foam, preferably neoprene foam, and/or;
the connectors (33) are crimped connections.

7. Garment (4) according to any of the previous claims wherein each sensor-actuator pad (B, C, D) of the plurality of the sensor-actuator pads further comprises a thermal pad (C) comprising:
a polymeric fibre charged with conductive particles or a fabric with a conductive ink, and thermal pad connectors for connecting conductive elements of the thermal pad (C) to the electrical bus (41).

8. Garment (4) according to claim 7 wherein the conductive ink is silver ink or the conductive particles are carbon black particles.

9. Garment (4) according to any of the claims 7-8 wherein the fabric with a conductive ink comprises polyurethane on a surface arranged to face the skin.

10. Garment (4) according to claim 7 wherein the polymeric fibre comprise a sheath made of a combination of polyolefin and carbon material and a core made of polyolefin, in particular the polyolefin being polypropylene.

11. Garment (4) according to any of the previous claims 1-10 wherein each sensor-actuator pad (B, C, D) of the plurality of the sensor-actuator pads further comprises a compression pad (D) comprising a textile elastic band with a shape-memory material and a plurality of compression pad connectors for electrically connecting the compression pad (D) to the electrical bus (41).

12. Garment (4) according to claim 15 wherein the compression pad (D) further comprises a plurality of rivets for connecting the springs to a non-elastic textile fabric for limiting extension of the spring.

13. Garment (4) according to claims 11 and 12 wherein the shape-memory material is a fibre made of a shape-memory polymer or a shape-memory alloy.

14. Garment (4) according to any of the previous claims 1-13 comprising:
an electronic control device (E) for controlling the plurality of sensor-actuator pads (B, C, D);
a waist band for supporting the electronic control device (E).

15. Garment (4) according to claim 14 further comprising electrical connectors for electrically connecting the waist band to the electrical bus (41).

## Patentansprüche

1. Hautdichtes, elastisches Kleidungsstück (4) zur Übertragung eines oder mehrerer Reize auf die Haut, umfassend:
ein elastisches Gewebe (4a - 4d), aus dem das Kleidungsstück hergestellt ist;
einen elektrischen Bus (41), der über eine Längsnaht des Kleidungsstücks genäht ist;
eine Vielzahl von Sensor-Aktuator-Plättchen (B, C, D), die jeweils elektrisch mit dem genannten elektrischen Bus (41) verbunden sind;
wobei jedes Sensor-Aktuator-Plättchen der Vielzahl der Sensor-Aktuator-Plättchen ein Plättchen zur Elektrostimulation (B) umfasst, umfassend
eine Vielzahl von Textilelektroden (32) für den Kontakt mit der Haut;
einen Schaumstoff (31) zwischen dem Gewebe (4a - 4d) und der Vielzahl der Textilelektroden (32);
eine Vielzahl von Anschlüssen (33), um das Plättchen zur Elektrostimulation (B) mit dem elektrischen Bus (41) zu verbinden.

2. Kleidungsstück (4) nach dem vorangehenden Anspruch, wobei jede Textilelektrode (32) der Vielzahl der Textilelektroden eine leitende Beschichtung (33) auf einer der Haut zugewandten Oberfläche umfasst.

3. Kleidungsstück (4) nach Anspruch 2, wobei die leitfähige Beschichtung (33) umfasst:
ein leitfähiges Polymer, insbesondere Polydimethylsiloxan;
oder Materialien aus Kohlenstoff, insbesondere Ruß und/oder nanostrukturierter Kohlenstoff, ferner insbesondere Nanoröhrchen aus Kohlenstoff.

4. Kleidungsstück (4) nach einem der vorangehenden Ansprüche, wobei die Vielzahl der Textilelektroden (32) sind:
leitfähige Fäden, die auf ein Gewebe für die Plättchen zur Elektrostimulation (B) aufgenäht sind, wobei das Gewebe für die Plättchen zur Elektrostimulation (B) bevorzugt aus einem Gewirk besteht;
insbesondere mit 117 dtex bis 235 dtex.

5. Kleidungsstück (4) nach einem der vorangehenden Ansprüche, wobei die Vielzahl der Textilelektroden (32) ein Gewebe ist, umfassend eine leitfähige Tinte.

6. Kleidungsstück (4) nach einem der vorangehenden Ansprüche, wobei:
der Schaumstoff (31) ein synthetischer Schaumstoff ist, bevorzugt Neoprenschaum, und/oder;
die Anschlüsse (33) Crimpverbindungen sind.

7. Kleidungsstück (4) nach einem der vorangehenden Ansprüche, wobei jedes Sensor-Aktuator-Plättchen (B, C, D) der Vielzahl der Sensor-Aktuator-Plättchen ferner ein Wärmeleitplättchen (C) umfasst, umfassend:
eine mit leitfähigen Partikeln angereicherte Polymerfaser oder ein mit einer leitfähigen Tinte versehenes Gewebe sowie Anschlüsse für Wärmeleitplättchen, um leitfähige Elemente des Wärmeleitplättchens (C) mit dem elektrischen Bus (41) zu verbinden.

8. Kleidungsstück (4) nach Anspruch 7, wobei die leitfähige Tinte eine Silbertinte ist oder die leitfähigen Partikel Rußpartikel sind.

9. Kleidungsstück (4) nach einem der Ansprüche 7-8, wobei das Gewebe mit einer leitfähigen Tinte Polyurethan auf einer der Haut zugewandten Oberfläche umfasst.

10. Kleidungsstück (4) nach Anspruch 7, wobei die Polymerfaser eine Hülle aus einer Verbindung von Polyolefin und Kohlenstoffmaterial sowie einen Kern aus Polyolefin umfasst, wobei das Polyolefin insbesondere Polypropylen ist.

11. Kleidungsstück (4) nach einem der vorangehenden Ansprüche 1-10, wobei jedes Sensor-Aktuator-Plättchen (B, C, D) der Vielzahl der Sensor-Aktuator-Plättchen ferner ein Kompressionsplättchen (D) umfasst, das ein elastisches Textilband mit einem Formgedächtnismaterial und eine Vielzahl von Anschlüssen für die Kompressionsplättchen umfasst, um die Anschlüsse der Kompressionsplättchen (D) elektrisch mit dem elektrischen Bus (41) zu verbinden.

12. Kleidungsstück (4) nach Anspruch 15, wobei das Kompressionsplättchen (D) ferner eine Vielzahl von Nieten umfasst, um die Federn mit einem nicht elastischen Textilgewebe zur Begrenzung der Ausdehnung der Feder zu verbinden.

13. Kleidungsstück (4) nach Anspruch 11 und 12, wobei das Formgedächtnismaterial eine Faser aus einem Formgedächtnispolymer oder einer Formgedächtnislegierung ist.

14. Kleidungsstück (4) nach einem der vorangehenden Ansprüche 1-13, umfassend:
eine elektronische Steuereinrichtung (E) zur Steuerung der Vielzahl der Sensor-Aktuator-Plättchen (B, C, D);
ein Hüftband zur Aufnahme des elektronischen Steuergeräts (E).

15. Kleidungsstück (4) nach Anspruch 14 ferner umfassend elektrische Anschlüsse, um das Hüftband elektrisch an den elektrischen Bus (41) anzuschließen.

## Revendications

1. Vêtement élastique moulant (4) pour transmission d'un ou de plusieurs stimulus à la peau, comprenant :
un tissu élastique (4a - 4d) à partir duquel le vêtement est fait ;
un bus électrique (41) cousu par-dessus une couture longitudinale du vêtement;
une pluralité de coussinets capteurs-actuateurs (B, C, D), chacun étant électriquement accouplé audit bus électrique (41) ;
dans lequel chaque coussinet capteur-actuateur de la pluralité de coussinets capteurs-actuateurs comprend un coussinet d'électrostimulation (B) comprenant
une pluralité d'électrodes textiles (32) destinées à être en contact avec la peau ; une mousse (31) entre le tissu (4a - 4d) et la pluralité d'électrodes textiles (32) ; une pluralité de connecteurs (33) pour connecter le coussinet d'électrostimulation (B) au bus électrique (41).

2. Vêtement (4) selon la revendication précédente dans lequel chaque électrode textile (32) de la pluralité d'électrodes textiles comprend un revêtement conducteur (33) sur une surface disposée pour être face à la peau.

3. Vêtement (4) selon la revendication 2 dans lequel le revêtement conducteur (33) comprend un :
polymère conducteur, en particulier le polydiméthylsiloxane ;
ou des matériaux carbonés, en particulier le noir de carbone et/ou le carbone nanostructuré, plus particulièrement des nanotubes de carbone.

4. Vêtement (4) selon l'une quelconque des revendications précédentes dans lequel la pluralité d'électrodes textiles (32) sont :
des fils conducteurs cousus à un tissu de coussinet d'électrostimulation (B), le tissu de coussinet d'électrostimulation (B) étant préférablement une matière tricotée ;
en particulier ayant de 117 dtex à 235 dtex.

5. Vêtement (4) selon l'une quelconque des revendications précédentes dans lequel la pluralité d'électrodes textiles (32) est un tissu comprenant une encre conductrice.

6. Vêtement (4) selon l'une quelconque des revendications précédentes dans lequel:
la mousse (31) est une mousse synthétique, préférablement une mousse néoprène, et/ou ;
les connecteurs (33) sont des connexions serties.

7. Vêtement (4) selon l'une quelconque des revendications précédentes dans lequel chaque coussinet capteur-actuateur (B, C, D) de la pluralité de coussinets capteurs-actuateurs comprend également un coussinet thermique (C) comprenant :
une fibre polymère chargée de particules conductrices ou un tissu avec une encre conductrice, et des connecteurs de coussinet thermique pour connecter des éléments conducteurs du coussinet thermique (C) au bus électrique (41).

8. Vêtement (4) selon la revendication 7 dans lequel l'encre conductrice est de l'encre argent ou les particules conductrices sont des particules de noir de carbone.

9. Vêtement (4) selon l'une quelconque des revendications 7-8 dans lequel le tissu avec une encre conductrice comprend du polyuréthane sur une surface disposée pour être face à la peau.

10. Vêtement (4) selon la revendication 7 dans lequel la fibre polymère comprend une gaine faite d'une combinaison de polyoléfine et de matériau carboné et un noyau fait de polyoléfine, la polyoléfine étant en particulier du polypropylène.

11. Vêtement (4) selon l'une quelconque des revendications précédentes 1-10 dans lequel chaque coussinet capteur-actuateur (B, C, D) de la pluralité de coussinets capteurs-actuateurs comprend également un coussinet de compression (D) comprenant une bande élastique textile avec un matériau à mémoire de forme et une pluralité de connecteurs de coussinet de compression destinés à connecter électriquement le coussinet de compression (D) au bus électrique (41).

12. Vêtement (4) selon la revendication 15 dans lequel le coussinet de compression (D) comprend également une pluralité de rivets destinés à connecter les ressorts à un tissu textile non élastique pour limiter l'extension du ressort.

13. Vêtement (4) selon les revendications 11 et 12 dans lequel le matériau à mémoire de forme est une fibre faite de polymère à mémoire de forme ou d'un alliage à mémoire de forme.

14. Vêtement (4) selon l'une quelconque des revendications précédentes 1-13 comprenant :
un dispositif de contrôle électronique (E) destiné à contrôler la pluralité de coussinets capteurs-actuateurs (B, C, D) ;
une ceinture pour tenir le dispositif de contrôle électronique (E).

15. Vêtement (4) selon la revendication 14 comprenant également des connecteurs électriques destinés à connecter électriquement la ceinture au bus électrique (41).
